Europäisches Patentamt

European Patent Office

Office européen des brevets

(11) Publication number:

**0 079 086**

**B1**

## (12) EUROPEAN PATENT SPECIFICATION

(45) Date of publication of patent specification: **01.07.87**

(21) Application number: **82110373.6**

(22) Date of filing: **10.11.82**

(51) Int. Cl.⁴: **G 01 D 3/02, A 61 B 5/02**

(54) Catheter sensor and memory unit.

(30) Priority: **10.11.81 NL 8105084**
**05.11.82 US 439517**

(43) Date of publication of application:
**18.05.83 Bulletin 83/20**

(45) Publication of the grant of the patent:
**01.07.87 Bulletin 87/27**

(84) Designated Contracting States:
**DE FR GB IT**

(56) References cited:
**EP-A-0 010 762**
**EP-A-0 070 674**
**GB-A-1 532 362**
**GB-A-2 065 890**
**US-A-4 240 441**
**US-A-4 274 423**

**POLYTECHNISCH TIJDSCHRIFT:**
**ELEKTROTECHNIEK/ELECTRONICA, vol. 35,**
**no. 9, September 1980, page 537, Den Haag**
**(NL); "Tip-drukmeetcatheter".**

The file contains technical information
submitted after the application was filed and
not included in this specification

(73) Proprietor: **Sentron v.o.f.**
**P.O. Box 38**
**NL-9301 LJ Roden (NL)**

(72) Inventor: **Meinema, Ate J.**
**Schonauwen 35**
**NL-9301 SN Roden (NL)**

(74) Representative: **Kuhnen, Wacker & Partner**
**Schneggstrasse 3-5 Postfach 1729**
**D-8050 Freising (DE)**

Courier Press, Leamington Spa, England.

## Description

The present invention relates to a sensor system and more specifically to a catheter sensor system for monitoring a chemical, physical or biological parameter and for providing a standard output. The system includes a non-uniform sensor for example in a catheter and a memory connected thereto and containing information data which is supplied in parallel with the sensor output to signal conditioning and processing circuits to produce a standard output for the parameter(s) sensed.

Sensors or sensor combinations that provide an electrically processable output signal are used in many fields for measuring and/or detecting a variety of phenomena. These phenomena may, for example, be of a chemical, physical or biological nature. As used herein, the term sensor can include a combination of cooperating sensors.

In the mass manufacture of sensors, an almost unavoidable problem is encountered in that, from one specimen sensor to another, the sensors have slightly different properties and exhibit a different behavior. This renders it difficult to compare accurately the results of measurements made with different sensors.

This drawback has been overcome in some instances by either applying very high standards in the production of the sensors, carrying out a very strict selection after production, and/or calibrating each sensor before use. These methods are time consuming, either on the side of production or on that of the consumer, and expensive.

A user of a sensor who is aware of the problem of the different response characteristics of different sensors will try to use the same sensor all the time after it has been calibrated. This, however, is often impossible in the field of medical sensors since certain sensors can only be used once and/or have to be replaced after several uses thereof to avoid infection to a patient which can occur if a sensor is sterilized more than a few times.

Also, heretofore, a sensor which can be easily calibrated before use has not been readily available.

Heretofore, attempts have been made to develop an "ideal" sensor in order to eliminate the above drawbacks. However, it is practically impossible to produce an "ideal" or perfect sensor and this is especially so in the case of sensors which must be mass produced in larger numbers.

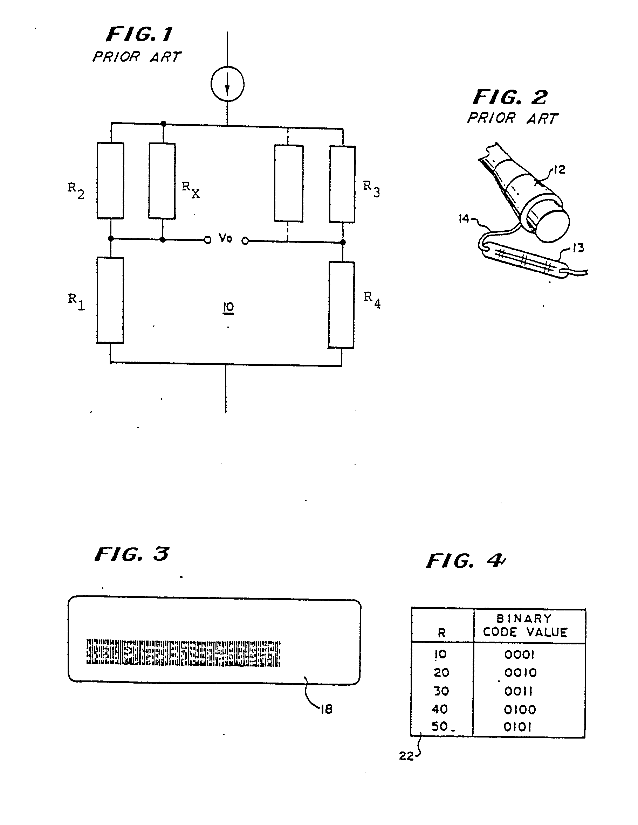

Additionally, it has been proposed to provide each sensor with an identification plate, chained, for example, to the sensor, and showing some characteristics of the sensor. The user can then correct the sensor's output signals by means of the data on the identification plate. Such correction may be effected, for example, by adjusting an electrical circuit arrangement which processes the sensor signals. This can be done, for example, by adjusting a potentiometer or thumb-wheel switches to effect the desired correction in the electrical circuit. This method is used, for example, in Fleisch Flow Transducers. (cp. Figure 2).

However, this method is not always effective since errors may occur in correlating a plate with a sensor, both during production, because, for example, the identification plates may be interchanged, and in use, because an identification plate is misread or the electrical circuit is maladjusted.

Such interchange of identification plates is not an uncommon event and periodically occurs in the mass production of sensors.

Furthermore, the adjustment of an electrical circuit by hand is a cumbersome job and lowers the market appeal of such sensors provided with identification plates.

In some sensor applications, such as, for example, in the medical field, it is of great importance that the risk of error be as low as possible. As a result, the manual adjustment of equipment coupled to sensors on the basis of data on an identification plate is highly undesirable.

Furthermore, even if manufacturing techniques are perfected to such an extent that certain types of sensors can be made sufficiently "ideal", it is yet often desirable to record specific unique information associated with a particular sensor in such a manner that when the sensor is used such unique information is immediately available without the risk of errors.

Such unique information may comprise, for example, the type of sensor, or type number, serial number, date of production, or safe use life of the sensor.

Consequently, an identification plate sensor combination as described above although useable in the medical field, still has the inherent drawbacks described above.

GB—A—2,065,890 entitled Sensor System with Non-linearity Correction from which the present invention starts in the precharacterizing part of Claim 1 proposes to provide a sensor system comprising a fluid tight housing having a fluid pressure inlet portion and the sensor being coupled to an electronic component housing, said housing including sensor amplifying circuits, a power supply, a transistor switch, a shift register and a PROM. A signal isolation interface circuit is fixed to one side of the electronic component housing for connecting the electrical component housing and electronic circuitry therein to a microprocessor.

The PROM in this sensor system is programmed with correction control data to compensate for non-linearity and/or perhaps, other characteristics in the sensor output.

As will be described in greater detail hereinafter, the sensor and memory unit of the present invention differ from the sensors or sensor systems described above by providing an integral, unitary sensor and memory system where information regarding the characteristics of the sensor or sensor-memory combination are permanently recorded in the memory and the sensor and memory are permanently coupled together. The recorded information can be automatically and directly read and retrieved by separate electronic processing circuitry.

The sensor system of the present invention is preferably employed in a catheter for carrying the sensor therein, such as at one end thereof, and for providing a conduit for wire conductor connections between the sensor or sensors in the catheter and the memory fixed to the catheter. The sensor and memory unit is disposable and is therefor particularly adapted for use in the medical field.

More specifically with respect to the sensor system disclosed in GB—A—2,065,890 such sensor system provides a method for correcting an output signal of an electronic signal conditioning circuit where memory information may be used for non-ideal transducer characteristics, such as non-linearity, which is reflected in the output signal of the electronic signal conditioning circuit. However, an ideal linear transducer may have sensitivity deviation from nominal sensitivity specifications and GB—A—2,065,890 does not indicate how these differences in sensitivity from nominal specifications can be treated.

As described in further detail hereinafter the sensor system of the present invention is utilized in a system where an ideal transducer is assumed, i.e., a perfectly linear transducer. However, all sensors built deviate from the nominal specification established therefor. In the memory of the present invention the actual specification of the ideal transducer (for example, pressure sensitivity, offset, temperature sensitivity) are stored. The sensor system of the present invention as claimed in Claim 1 includes signal processing means that can be coupled to the sensor and memory unit; information data stored in the memory is decoded and the characteristics of the signal conditioning circuitry associated with the microprocessor are changed (for example, the amplification factor or offset are changed). As a result, the output signal from the signal conditioning circuitry always will be the same as for a sensor or transducer with nominal specification for any sensor and memory unit that is coupled into the sensor system of the present invention. Stated otherwise, the final output signal is standard for every transducer whereas in the sensor system disclosed in GB—A—2,065,890, the amplitude of the signal depends on transducer sensitivity.

Also the memory can contain direct data for the adjustment of the signal processing and conditioning circuitry instead of data concerning the sensor characteristics.

Finally, and what has been explained above, the sensor and memory unit is just that, namely a sensor and memory unit alone without any signal processing and conditioning circuitry, so as to provide a simple, single and compact unit which can be incorporated into a catheter for use in the medical field and which can be detachably coupled to any one of several types of signal processing and conditioning circuits.

Because of the simple and thus inexpensive construction the sensor and memory unit is disposable and is thus particularly useful for medical applications.

From EP—A—0070674 published on January 26, 1983 a connector for a thermodilution catheter is known comprising a plurality of connection pins forming a kind of matrix. By means of said matrix the catheter size is communicated to a computer for adjusting same.

The following is a description of preferred embodiments taken in conjunction with the accompanying drawings, in which:

Figure 1 is a schematic circuit diagram of a prior art sensor incorporated into a Wheatstone bridge;

Figure 2 is a perspective view of a prior art sensor provided with an identification plate;

Figure 3 is a plan view of a bar code defining the memory of one embodiment of a sensor and memory unit;

Figure 4 is a resistance/binary table defining the memory of another sensing and memory unit;

Figure 5 is a perspective view of still another cathether sensor and memory unit;

Figure 6 is a sectional view of the sensor assembly at the end of the catheter shown in Figure 5;

Figure 7 is a sectional view of the memory module coupled to the catheter shown in Figure 5;

Figure 8 is a sectional view of the memory module shown in Figure 7 and is taken along line 8—8 of Figure 7;

Figure 9 is a plan view of an in-line pressure sensor, catheter and memory unit of yet another embodiment of the present invention;

Figure 10 is a schematic circuit diagram of one type of signal processing and conditioning circuit to which a sensor and memory unit can be detachably coupled to form a sensor system of the present invention;

Figure 11 is a schematic circuit diagram of another type of signal processing and conditioning circuit to which a sensor and memory unit can be detachably coupled to form a sensor system of the present invention; and

Figure 12 is a schematic circuit diagram of still another type of signal processing and conditioning circuit to which a sensor and memory unit can be detachably coupled to form a sensor system of the present invention.

Referring now to the drawings in greater detail there is illustrated in Figure 1 a Wheatstone bridge 10 comprising four resistors $R_1$ to $R_4$. These resistors form a sensor 10 which, for example, may be a pressure sensitive sensor. Such circuit arrangements are well known.

According to known and conventional methods of production, an output voltage Vo is measured, and one or more suitable resistors $R_x$ are selected and mounted in the bridge 10, in order that bridge equilibrium and the sensitivity of the sensor 10 constituted by the bridge 10 may be adjusted in any desired way.

In this embodiment a series of sensors 10 are produced which, it is true, are all equal to the extent possible, but whose sensitivity are generally that of the sensor 10 having the lowest sensitivity.

The individual adjustment of each sensor 10 is expensive and time consuming, and in addition requires a large stock of resistors for the resistor $R_x$.

In Figure 2 is illustrated a sensor 12 provided with an identification plate 13 which in the case shown is attached to the sensor by a cord or chain 14. The sensor 12 can be a Fleisch Flow Transducer. In the manufacture of such sensors 12, no effort is made to produce standard sensors 12. Instead, correction of the sensor 12 characteristics is done by the user, who adjusts equipment (not shown) for processing the output signals from a particular sensor 12 on the basis of the data carried by the identification plate 13 therefor. The disadvantages of this method of correction have been described above.

According to the teachings of the present invention, no attempt is made to produce sensors as uniform as possible by trimming or other adjustment. On the contrary, the characteristic properties of each sensor with regard to ambient effects, sensitivity and the like are measured. Such a measurement has been conventional in the past for purposes of quality control.

Further according to the teachings of the present invention, the resulting measurements and/or other data uniquely associated with the sensor are not specified on an identification plate, but joined with the sensor in such a manner that it is automatically processed by the equipment coupled to the sensor without any initial adjusting operations being required from the user.

For this purpose, the measurement data and/or other data are stored in a memory, the contents of which can be processed automatically and electronically. The memory is permanently connected to the associated sensor. For this purpose the memory may be incorporated, for example, in the sensor housing or in the sensor's connector. It is conceivable that, for certain uses, the memory is constituted by a specific configuration of, or specific connection of, connector pins, or by a specific mechanical treatment of the connector housing.

A special configuration, of connector pins can be realized, for example, by placing the pins in a specific pattern and/or machining one or more pins in a house-key fashion.

The processing equipment then will include a complementary connector suitable for all possible configurations, and connected in such a manner that the information stored in the connector is processed in the proper manner. Alternatively, means for detecting the mechanical treatment of the connector housing are provided.

Moreover, a suitable memory can be realized, for example, by a magnetic card, a bar code marked on a carrier, a resistance code, or a PROM (programmable read only memory).

The processing equipment must, of course, be adapted to the type of memory being used. Such an adaptation, however, should not present any problems to those skilled in the art.

In Figure 3 is shown an example of a bar code 18 embodying the characteristics of a sensor with which it is associated and for which it constitutes the memory of one embodiment of a sensor system constructed according to the teachings of the present invention. Such a bar code 18 is preferably marked on a connector housing, e.g., printed, engraved or impressed.

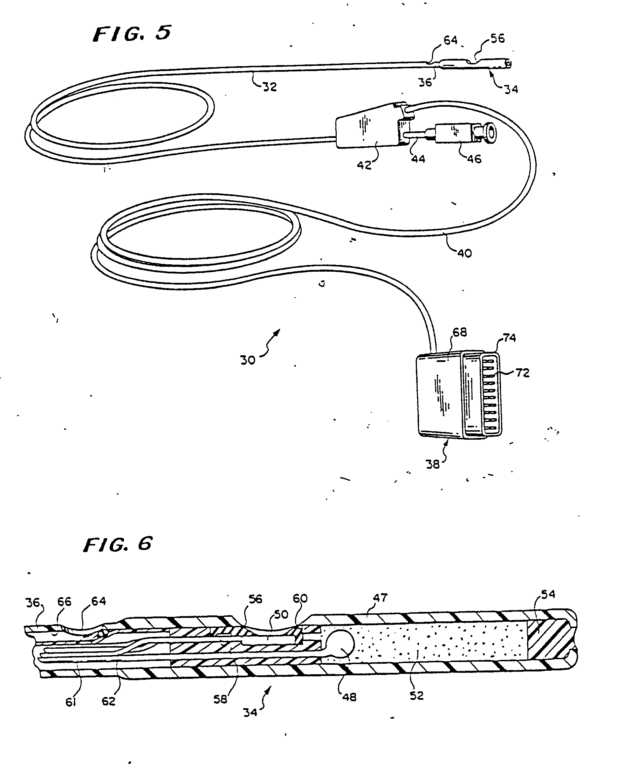

In Figure 4 is shown a table 22 of resistance/binary code. Each resistor or combination of resistors R corresponds to a binary number specified in the table 22 stored in processing equipment. The processing equipment may comprise a microprocessor, which, under the control of the binary number associated with a given resistance code, effects certain corrections with regard to the sensor signal. The resistance/binary code may not be adequate, however, if a substantial amount of data concerning the sensor is to be stored, since large quantities of resistors must be kept in stock. For minimal data, however, the resistance code is quite suitable.

The use of a PROM as a memory appears to be very suitable. PROM's are commercially available with very small dimensions but with a relatively large memory capacity of 256 bits or more.

A PROM suitable for the purposes of the present invention is, for example, the IM 5600—5610 series of Intersil, Inc.

During the production of sensors, a PROM can be automatically programmed by a computer-controlled testing system as used in the production of large numbers.

Furthermore, as stated before, the type of sensor and other unique information, such as type number, serial number, production date, safe service life, etc. can be recorded in the memory, which offers the possibility of designing processing equipment suitable for various types of sensors.

In this way, there is provided with the sensor and memory in conjunction with a deciphering (microprocessor) circuit and signal conditioning circuit a multi-purpose measuring and detecting system which requires no user calibration thus enhancing its consumer appeal and which minimizes, if not altogether eliminates user errors in calibration and use. Also such a sensor system can readily be combined with a catheter to provide one preferred embodiment of the present invention, namely a catheter sensor and memory unit 30 as will now be described in detail in connection with the following description of Figures 5—12.

As shown in Figure 5, the catheter sensor and memory unit 30 has a sensor assembly 34 at one end 36 of the catheter 32 and a memory module 38 is coupled by a multi-conductor lead 40 through a three way connector 42 to the other end 44 of the catheter 32. Also a coupling 46 is provided at the end 44 of the catheter 32 for coupling same to a fluid delivery or withdrawal system (not shown).

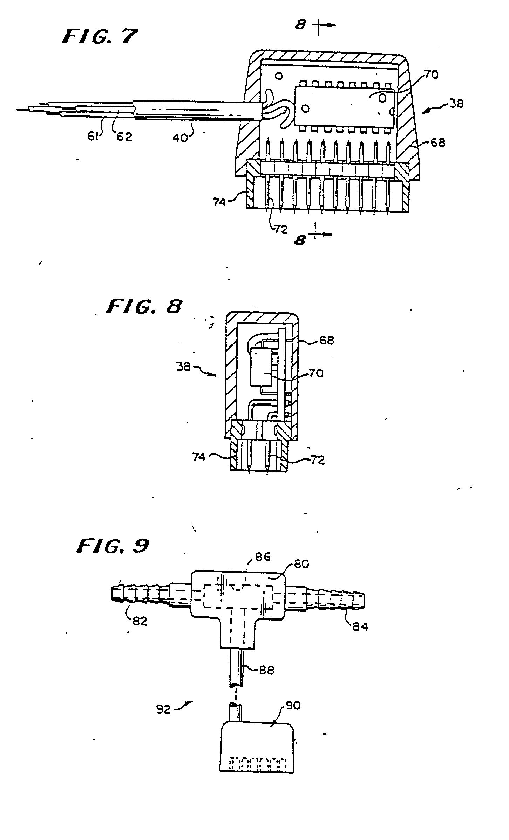

As shown in Figure 6, the sensor assembly 34 includes a tube 47 having two sensors 48 and 50 therein. The sensor 48 is a temperature sensor bulb and the sensor 50 can be a pressure sensor or an electrode for in vivo measurement of body fluids. The temperature sensor bulb 48 is surrounded by thermal conducting material 52 packed in the end of the tube 47 which is sealed off by an end plug 54.

The tube 47 has a hole 56 therein for sensing pressure or for measuring body fluids. In this respect, inside the tube 47 is a carrier 58 mounting the sensor or electrode 50 beneath the hole 56. A sealing material 60 is provided in the tube 47 around the sensor or electrode 50 and the hole 56.

Where body fluids are to be measured a membrane (not shown) formed of hydrogel can be positioned across the hole or aperture 56 so as to form an ion diffusion barrier between body fluids to be measured and electrolyte material within the tube 47 and in contact with electrode 50.

Several leads or insulated conductors such as conductor 61 from the temperature sensor bulb 48 and a conductor 62 from the sensor or electrode 50 extend rearwardly from the sensor assembly 34 through the catheter 32 to the three way connector 42 where the plurality of wire conductors 61 and 62 then branch off into the multiconductor cable 40 leading to the memory module 38.

In the distal end 36 of the catheter 32 there is provided an opening 64 which communicates with a passageway 66 within the catheter 32. This passageway 66 extends to and through the connector 42 through the proximal end 44 of the catheter 32 to the coupling 46 and facilitates the insertion or withdrawal of fluids through catheter 32 to or from the opening 64.

Where the sensor 50 is an electrode for measuring body fluids, the opening 64 can be positioned so as to open adjacent the membrane of hydrogel so that liquid exiting therefrom can provide a flushing action across the surface of the hydrogel membrane.

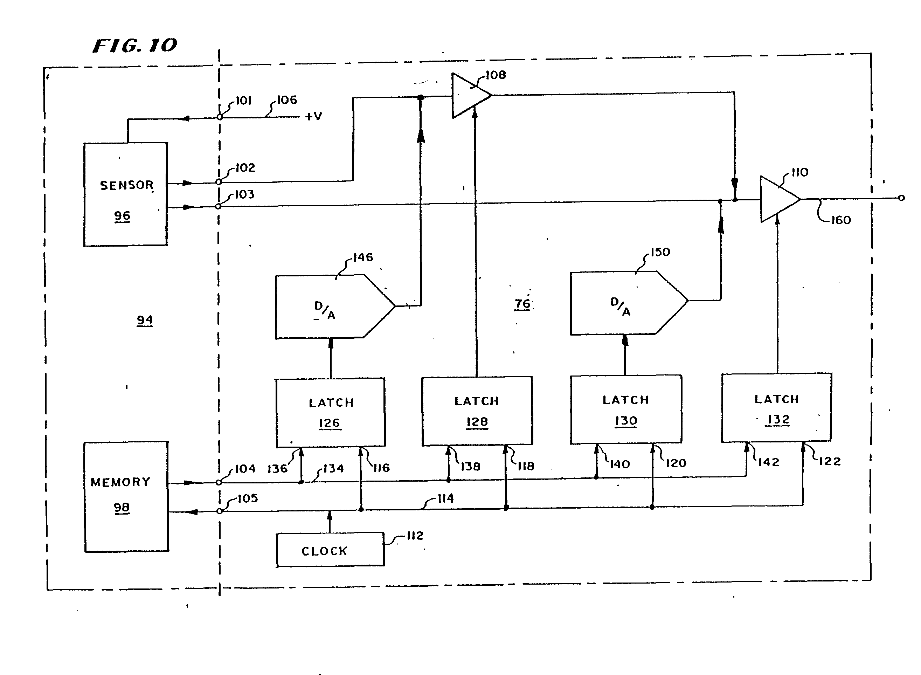

As shown in Figures 7 and 8, the multiconductor lead 40 extends to and into a housing 68 of the memory module 38. As shown, a memory 70 such as a PROM is mounted within the housing 68 on a circuitboard. Wire conductors such as conductors 61 and 62 within the multiconductor lead 40 connect the sensors 48 and 50 to connector pins 72 situated within a connector housing 74 fixed to the housing 68. The PROM 70 is also connected to the connector pins 72.

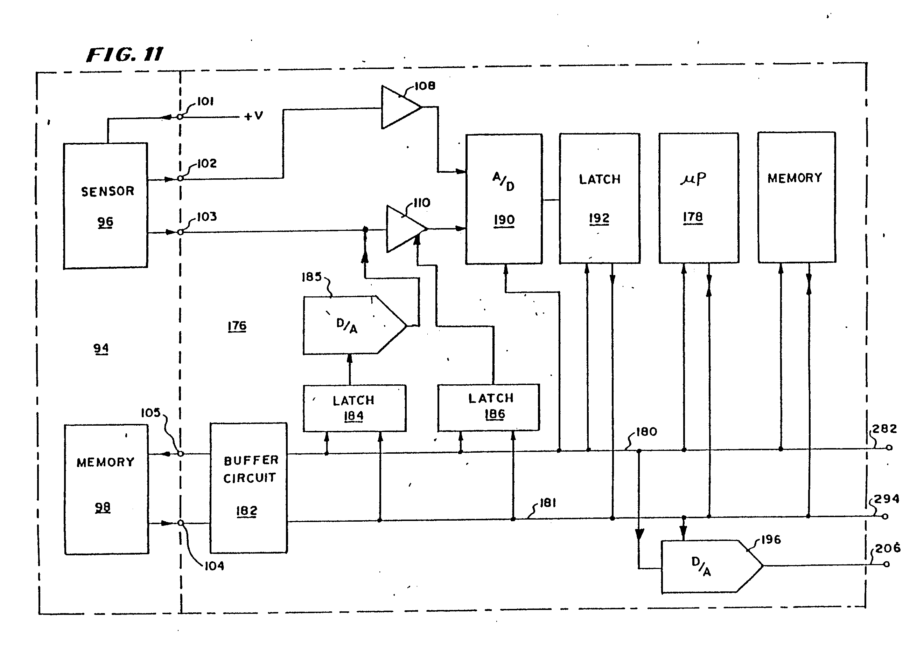

The connector pins 72 within connector housing 74 can then be easily connected to a mating connector socket module associated with signal processing and conditioning means 76 (Figure 10).

In Figure 9 is shown a three way coupling member or flow cell 80 which has in-line nozzles 82 and 84 on either side thereof and a sensing chamber 86 therein. The sensing chamber 86 has at least one sensor therein which is coupled by a lead 88 to a memory module 90 similar to or identical with the memory module 38 to form another embodiment of a sensor and memory unit 92 constructed in accordance with the teachings of the present invention. The sensor can be a flow rate sensor or a pressure sensor.

Referring now to Figure 10 there is illustrated therein a signal processing and conditioning circuit 76 (hereinafter referred to as "processing means") which is coupled to a sensor and memory unit 94 to constitute a sensor system constructed in accordance with the teachings of the present invention and which for example can be the catheter sensor and memory unit shown in Figure 5.

The sensor and memory unit 94 includes a sensor 96 and a memory 98. The processing means 76 is adapted to be coupled by means of connector pins 101 to 105 to the sensor and memory unit 94 as shown. Processing means 76 is adapted to receive and detect the characteristic data in the memory 98 associated with the sensor 96 and to process such data.

The data is typically stored in digital form is the memory 98 associated with the sensor 96 and is converted in the processing means 76 into voltages, currents or gain factors and such voltages, currents or gain factors are utilized in adjusting circuit components in the processing means 76.

If the sensor and memory unit 94 is of the type illustrated in Figure 5, the sensor 96 is housed or mounted within the distal end of a catheter. Electrical conductors within the catheter then connect the sensor 96 to connector pins 101 to 103 in a connector housing (not shown). The memory 98 associated with the sensor 96 can then be mounted in a connector housing (not shown) such as the housing 68 shown in Figure 5 and connected to connector pins 104 and 105 as shown.

A voltage bus 106 is connected to connector pin 101 for supplying voltage to the sensor 96. If necessary, such voltage can also be supplied to the memory 98.

The sensor 96 can be of the type which will provide a temperature sensor signal to connector pin 102 and a pressure sensor signal to pin 103 which pins 102 and 103 are connected, respectively, to a first amplifier 108 and a second amplifier 110. As shown, the output of the amplifier 108 which receives the temperature sensor signal is combined with the pressure sensor signal supplied to the second amplifier 110 for controlling the adjustment of the second amplifier 110. Here we have temperature dependent pressure sensor signals and pressure dependent temperature sensor signals.

The adjustments of the amplifiers 108 and 110 are further controlled by the data read from the memory 98 and supplied to connector pin 104. This adjustment is effected by a clock 112. In this respect, the clock 112 is coupled via a bus 114 to the terminal pin 105 for supplying a clock pulse to the memory 98 connected to the connector pins 104 and 105.

As shown, the clock 112 supplies a clock pulse to the bus 114 which then supplies the clock pulse to the memory 98 and to clock inputs 116, 118, 120 and 122 of latching circuits 126, 128, 130 and 132. The data supplied to the connector pin 104 from the memory 98 is placed on a bus 134 which is connected to data inputs 136, 138, 140 and 142 of the latching circuits 126, 128, 130 and 132. As a result, each time a clock pulse is outputted by the clock 112, the data on the bus 134 from the memory is inputted to the respective latching circuits 126, 128, 130 and 132.

The latching circuits 128 and 132 then output a gain factor signal to the amplifiers 108 and 110 respectively as shown.

In a similar manner, the correction data clocked into the latches 126 and 130 are outputted to respective digital to analog converter circuits 146 and 150 which then output a bias or offsetting signal to the input of amplifiers 108 and 110 respectively as shown.

A conditioned analog sensor signal then ultimately appears at output 160 of the second amplifier 110 and can be processed further in a suitable manner.

In Figure 11 there is shown a different type of processing means 176 which can be coupled to the sensor and memory unit 94 as shown. Means 176 includes a microprocessor 178 which is coupled by an address bus 180 and data bus 181 to a buffer circuit 182 that is connected to connector pins 104 and 105 for retrieving information data from the memory 98. The microprocessor 178 processes the data retrieved from the memory 98 and then causes appropriate adjustment of the second amplifier 110. In this respect, the microprocessor 178 supplies address signals to the address bus 180 which controls latch circuits 184 and 186. Latch circuit 184 supplies a digital signal to a D/A converter 185 which outputs an analog signal that is input to second amplifier 110 as an offset signal. The latch 186 supplies a gain adjust or gain factor signal directly to the second amplifier 110 as shown. The output signals from the amplifiers 108 and 110 are then digitized by an A/D converter 190 that has an output coupled to another latch circuit 192.

The microprocessor 178 will cause the latch circuit 192 to output a corrected digital sensor signal to the data bus 181. A D/A converter 196 is coupled to the address bus 180 and the data bus 181 and is operated by the microprocessor 178 to output at output 206 a conditioned analog sensor signal converted from the signal supplied to the data bus 181 by the latch 192.

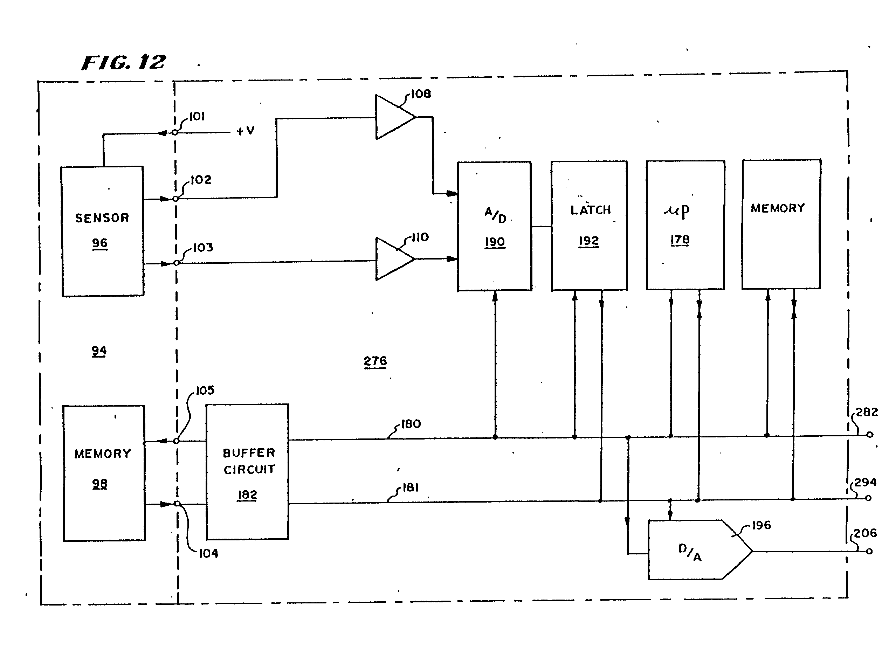

Referring now to Figure 12 there is illustrated therein a modified processing means 276 which is similar to means 176 except that in means 276 the latches 184 and 186 and the D/A converter 185 are omitted and the amplifiers 108 and 110 have a fixed amplification setting. In this modified processing means, the data stored in the memory 98 is not used by the microprocessor 178 for adjusting the settings of amplifier 110. Rather, the data stored are used as computational magnitudes for processing the sensor signal.

The information stored in the memory 98 can be utilized to process the sensor signals supplied to the amplifiers 108 and 110. Alternatively, the information data in the memory 98 can be passed directly to output terminals 282 and 294 as shown in Figures 11 and 12, after conversion, if necessary, into a form suitable for further processing, such as for example, for being displayed on a display device (not shown).

It will be understood from the foregoing description that the teachings of the present invention can be utilized in different ways in a method for manufacturing sensors or sensor combinations together with the recording of characteristic data for individual sensor or sensor combinations in an automatically and directly electrically readable permanent memory. Also according to this method, the memory is permanently connected to the sensor or sensor combination.

Obviously, the particular data stored, the magnitude to be corrected and the number of corrections will depend, of course, upon the particular sensor utilized. In this respect, the sensor or sensor combination can include a flow rate sensor, a pressure sensor, a temperature sensor, an electrolyte sensor or a chemical sensor.

Moreover, modifications can be made to the various embodiments of the present invention described above without departing from the teachings of the invention. For example, the chamber 86 within the three way coupling member 80 can have a flow rate sensor mounted therein instead of a pressure sensor.

Specifically, the sensor and memory unit of the present invention is disposable, can be mass produced, and can be utilized in the medical field or in the airplane industry.

**Claims**

1. A sensor system comprising:
at least one sensor (48, 50; 96) said at least one sensor providing an electrically processable output signal; memory means (18, 22, 70; 90; 98) arranged at said at least one sensor and containing information data for the conditioning of the sensor output signal; processing means (76, 176; 276) connected with said sensor for processing and conditioning the sensor output signal; means (112, 182) for reading out said information data from said memory means; and means for controlling the sensor output signal in dependency upon the information recorded in the memory means to obtain a sensor system output signal, characterized in that said memory means (18, 22, 70; 90; 98) is permanently connected to said at least one sensor (48, 50; 96)

said sensor being a disposable sensor; and

said processing means (76, 176; 276) takes the output signals from the sensor and processes them to a standard output signal by a first signal path (108, 110) in said processing means (76, 176; 276) and by a parallel second signal path (114, 134; 180, 181) which comprises means for detecting and processing the information recorded in the memory means (18, 22, 70; 90; 98).

2. The system of Claim 1 characterized in that said at least one sensor (48, 50; 96) is mounted in a housing (47) and said memory means (18, 22; 70) is also mounted in a housing (38; 90).

3. The system of Claim 1 characterized by comprising means (68, 72, 74) for connecting said at least one sensor (48; 50) to said processing means (76, 176; 276) for processing the sensor signals, and by said memory means (70; 98) being carried by the connecting means (68, 72, 74).

4. The system of Claim 1 characterized in that said memory means (70; 98) is a PROM.

5. The system of Claim 1 characterized in that said memory means (70) is mounted in a memory module (38; 90) including a connector housing (74) having connector pins (72) mounted therein for facilitating coupling of said at least one sensor (48; 50; 96) to the signal processing means (76, 176; 276).

6. The system of Claim 1 characterized in that said memory means (70; 98) is mounted in a housing (68) and said housing (68) is permanently connected by a cable (40; 88) to said at least one sensor (48, 50).

7. The system of Claim 1 characterized in that said at least one sensor is mounted in a flow cell (86).

8. The system of Claim 7 characterized in that said flow cell (86) includes a housing (80) having a chamber therein and in-line nozzles (82, 84) extending from opposite ends of said housing (80) for being series connected in a fluid circuit.

9. The system of any one of Claims 1 to 7 characterized in that said at least one sensor (48, 50) is mounted in a catheter (32).

10. The system of Claim 9 characterized in that said at least one sensor (48, 50) is mounted at the distal end (36) of said catheter (32).

11. The system of Claim 10 characterized by including a three way coupling device (42) mounted to said catheter (32) adjacent the proximal end (44) thereof, a memory module (38) having said memory means (70) therein and being connected to said three way coupling device (42) by a multiconductor lead (40) having conductors (61, 62) therein which extend through the coupling device (42) and said catheter (32) to said at least one sensor (48, 50), and a passage means (66) extending through the coupling device (42) and the proximal end (44) of said catheter to a fluid coupling means (46).

12. The system of Claim 9 characterized in that said at least one sensor (48, 50) is a flow rate sensor.

13. The system of Claim 9 characterized in that said at least one sensor (48, 50) is a pressure sensor.

14. The system of Claim 9 characterized in that said at least one sensor (48, 50) is a temperature sensor (48).

15. The system of Claim 9 characterized in that said at least one sensor (48, 50) includes a body fluid sensing electrode (50) for making electrolyte measurements.

16. The system of Claim 9 characterized in that said at least one sensor (48, 50) is a sensor (50) for making chemical measurements.

17. The system of any one of Claims 1 to 16 characterized in that said signal processing means (76, 176; 276) includes a microprocessor (178) for detecting and processing the data recorded in said memory means (98) in order to control the transmission function of the processing means by the first signal path (108, 110) in dependency upon the data retrieved from said memory means (98) for processing the sensor signals and for supplying the conditioned sensor signals to an output terminal (206).

**Patentansprüche**

1. Sensorsystem mit:

wenigstens einem Sensor (48, 50; 96), wobei der wenigstens eine Sensor ein elektrisch verarbeitbares Ausgangssignal erzeugt; Speichereinrichtungen (18, 22, 70; 90; 98), die im Bereich des wenigstens einen Sensors angeordnet sind und Informationsdaten beinhalten, um das Sensorausgangssignal zu beeinflussen; Verarbeitungseinrichtungen (76, 176; 276), welche mit dem Sensor verbunden sind, um das Sensorausgangssignal zu verarbeiten und zu beeinflussen; Einrichtungen (112, 182) zum Auslesen der Informationsdaten aus den Speichereinrichtungen; und Einrichtungen zur Steuerung des Sensorausgangssignals in Abhängigkeit der in den Speichereinrichtungen gespeicherten Informationen, um ein Sensorsystem-Ausgangssignal zu erhalten, dadurch gekennzeichnet,

daß die Speichereinrichtungen (18, 22, 70; 90; 98) permanent mit dem wenigstens einen Sensor (48, 50; 96) verbunden sind;

daß der Sensor ein Wegwerf-Sensor ist; und

daß die Verarbeitungseinrichtungen (76, 176; 276) die Ausgangssignale von dem Sensor übernehmen und sie in ein Standard-Ausgangssignal über einen ersten Signalpfad (108, 110) in den Verarbeitungseinrichtungen (76, 176; 276) und über einen parallelen zweiten Signalpfad (114, 134; 180, 181) umwandeln, der Einrichtungen zur Erkennung und Verarbeitung der in den Speichereinrichtungen (18, 22, 70; 90; 98) gespeicherten Informationen aufweist.

2. System nach Anspruch 1, dadurch gekennzeichnet, daß der wenigstens eine Sensor (48, 50; 96) in einem Gehäuse (47) angeordnet ist und die Speichereinrichtungen (18, 22; 70) ebenfalls in einem Gehäuse (38; 90) angeordnet sind.

3. System nach Anspruch 1, dadurch gekennzeichnet, daß Einrichtungen (68, 72, 74) vorgesehen sind, um den wenigstens einen Sensor (48; 50) mit den Verarbeitungseinrichtungen (76, 176; 276) zu verbinden, um die Sensorsignale zu verarbeiten, wobei dei Speichereinrichtungen (70; 98) von den Verbindungseinrichtungen (68, 72, 74) getragen werden.

4. System nach Anspruch 1, dadurch gekennzeichnet, daß die Speichereinrichtungen (70; 98) aus einem PROM bestehen.

5. System nach Anspruch 1, dadurch gekennzeichnet, daß die Speichereinrichtungen (70) in einem Speichermodul (38; 90) angeordnet sind, welches ein Verbindungsgehäuse (74) mit Verbindungsstiften (72) darin aufweist, um die Verbindung des wenigstens einen Sensors (48, 50; 96) mit den Signal-Verarbeitungseinrichtungen (76, 176; 276) zu erleichtern.

6. System nach Anspruch 1, dadurch gekennzeichnet, daß die Speichereinrichtungen (70; 98) in einem Gehäuse (68) angeordnet sind und das Gehäuse (68) permanent mittels eines Kabels (48; 88) mit dem wenigstens einen Sensor (48, 50) verbunden ist.

7. System nach Anspruch 1, dadurch gekennzeichnet, daß der wenigstens eine Sensor in einer Strömungszelle (86) angeordnet ist.

8. System nach Anspruch 7, dadurch gekennzeichnet, daß die Strömungszelle (86) ein Gehäuse (80) mit einer Kammer darin und in einer Linie liegende Nasen (82, 84) aufweist, welche sich von einander gegenüberliegenden Enden des Gehäuses (80) erstrecken, um in einem Strömungskreis in Reihe geschaltet zu werden.

9. System nach einem der Ansprüche 1—7, dadurch gekennzeichnet, daß der wenigstens eine Sensor (48, 50) in einem Katheter (32) angeordnet ist.

10. System nach Anspruch 9, dadurch gekennzeichnet, daß der wenigstens eine Sensor (48, 50) am distalen Ende (36) des Katheters (32) angeordnet ist.

11. System nach Anspruch 10, gekennzeichnet, durch: eine Dreiweg-Verbindungsvorrichtung (42), welche an dem Katheter (32) nahe seines proximalen Endes (44) angeordnet ist; ein Speichermodul (38), welches die Speichereinrichtungen (70) beinhaltet und mit der Dreiweg-Verbindungsvorrichtung (42) über einen Mehrfachleiter (40) verbunden ist; der Leiter (61, 62) aufweist, welche sich durch die Verbindungsvorrichtung (42) und dem Katheter (32) zu dem wenigstens einen Sensor (48, 50) erstrecken und eine Durchlaßeinrichtung (66), welche sich durch die Verbindungsvorrichtung (42) und das proximale Ende (44) des Katheters zu einer Fluid-Verbindungsvorrichtung (46) erstreckt.

12. System nach Anspruch 9, dadurch gekennzeichnet, daß der wenigstens eine Sensor (48, 50) eine Flußraten-Sensor ist.

13. System nach Anspruch 9, dadurch gekennzeichnet, daß der wenigstens eine Sensor (48, 50) ein Drucksensor ist.

14. System nach Anspruch 9, dadurch gekennzeichnet, daß der wenigstens eine Sensor (48, 50) ein Temperatursensor (48) ist.

15. System nach Anspruch 9, dadurch gekennzeichet, daß der wenigstens eine Sensor (48, 50) eine Körperflüssigkeit-Fühlelektrode (50) zur Durchführung elektrolytischer Messungen aufweist.

16. System nach Anspruch 9, dadurch gekennzeichnet, daß der wenigstens eine Sensor (48, 50) ein Sensor (50) zur Durchführung chemischer Messungen ist.

17. System nach einem der Ansprüche 1—16, dadurch gekennzeichnet, daß die Signalverarbeitungseinrichtungen (76, 176; 276) einen Mikroprozessor (178) zur Erkennung und Verarbeitung der in den Speichereinrichtungen (98) gespeicherten Daten aufweisen, um die Übertragungsfunktion der Verarbeitungseinrichtungen über den ersten Signalpfad (108, 110) in Abhängigkeit der von den Speichereinrichtungen (98) erhaltenen Daten zu steuern, um die Sensorsignale zu verarbeiten und um die verarbeiteten Sensorsignale einem Ausgangsanschluß (206) zuzuführen.

**Revendications**

1. Système de détection comprenant: au moins un capteur (48, 50; 96), ledit au moins un capteur fournissant un signal de sortie qui peut être traité électriquement; des moyens de mémoire (18, 22, 70; 90; 98) prévus audit au moins un capteur et contenant des données d'information pour le conditionnement du signal de sortie du capteur; des moyens de traitement (76, 176; 276) reliés audit capteur pour traiter et conditionner le signal de sortie du capteur; des moyens (112, 182) pour extraire lesdites données d'information desdits moyens de mémoire; et des moyens de commande du signal de sortie du capteur en fonction de l'information enregistrée dans les moyens de mémoire, pour obtenir un signal de sortie du système de capteur, caractérisé en ce que lesdits moyens de mémoire (18, 22, 70; 90; 98) sont reliés en permanence audit au moins un capteur (48, 50; 96), ledit capteur étant un capteur jetable; et lesdits moyens de traitement (76, 176; 276) reçoivent les signaux de sortie du capteur et les traitent pour obtenir un signal de sortie standard suivant un premier chemin de signal (108, 110) dans lesdits moyens de traitement (76, 176; 276) et suivant un deuxième chemin de signal parallèle (114, 134; 180, 181) qui comprend des moyens pour détecter et traiter l'information enregistrée dans les moyens de mémoire (18, 22, 70; 90; 98).

2. Système suivant la revendication 1, caractérisé en ce que ledit au moins un capteur (48, 50; 96) est monté dans un boîtier (47) et lesdits moyens de mémoire (18, 22; 70) sont également montés dans un boîtier (38; 90).

3. Système suivant la revendication 1, caractérisé en ce qu'il comprend des moyens (68, 72, 74) de liaison dudit au moins un capteur (48; 50) auxdits moyens de traitement (76, 176; 276) pour traiter les signaux du capteur, et en ce que lesdits moyens de mémoire (70; 98) sont portés par les moyens de liaison (68, 72, 74).

4. Système suivant la revendication 1, caractérisé en ce que les moyens de mémoire (70; 98) sont une mémoire morte programmable (PROM).

5. Système suivant la revendication 1, caractérisé en ce que lesdits moyens de mémoire (70) sont montés dans un module de mémoire (38; 90) comprenant un boîtier de connecteur (74) dans lequel sont montées des broches de connexion (72) pour faciliter le raccordement dudit au moins un capteur (48, 50; 96) aux moyens de traitement de signal (76, 176; 276).

6. Système suivant la revendication 1, caractérisé en ce que lesdits moyens de mémoire (70; 98) sont montés dans un boîtier (68) et ledit boîtier (68) est relié de façon permanente par un câble (40; 88) audit au moins un capteur (48, 50).

7. Système suivant la revendication 1, caractérisé en ce que ledit au moins un capteur est monté dans une cellule à circulation (86).

8. Système suivant la revendication 7, caractérisé en ce que ladite cellule à circulation (86) comprend un boîtier (80) comportant une chambre intérieure et des tubulures en ligne (82, 84) qui partent des extrémités opposées dudit boîtier (80) pour être raccordées en série dans un circuit de fluide.

9. Système suivant l'une quelconque des revendications 1 à 7, caractérisé en ce que ledit au moins un capteur (48, 50) est monté dans un cathéter (32).

10. Système suivant la revendication 9, caractérisé en ce que ledit au moins un capteur (48, 50) est monté à l'extrémité distale (36) dudit cathéter (32).

11. Système suivant la revendication 10, caractérisé en ce qu'il comprend un dispositif de raccordement à trois voies (42) monté sur ledit cathéter (32) près de son extrémité proximale (44), un module de mémoire (38) contenant lesdits moyens de mémoire (70) et relié audit dispositif de raccordement à trois voies (42) par un câble multiconducteur (40) contenant des conducteurs (61, 62) qui passent à travers le dispositif de raccordement (42) et dans ledit cathéter (32) jusqu'audit au moins un capteur (48, 50), et un organe de passage (66) qui traverse le dispositif de raccordement (42) et passe dans l'extrémité proximale (44) dudit cathéter jusqu'à un organe de raccordement de fluide (46).

12. Système suivant la revendication 9, caractérisé en ce que ledit au moins un capteur (48, 50) est un capteur de débit.

13. Système suivant la revendication 9, caractérisé en ce que ledit au moins un capteur (48, 50) est un capteur de pression.

14. Système suivant la revendication 9, caractérisé en ce que ledit au moins un capteur (48, 50) est un capteur de température (48).

15. Système suivant la revendication 9, caractérisé en ce que ledit au moins un capteur (48, 50) comprend une électrode (50) de détection de fluide corporel pour effectuer des mesures d'électrolyte.

16. Système suivant la revendication 9, caractérisé en ce que ledit au moins un capteur (48, 50) est un capteur (50) pour effectuer des mesures chimiques.

17. Système suivant l'une quelconque des revendications 1 à 16, caractérisé en ce que lesdits moyens de traitement de signal 76, 176; 276) comprenent un microprocesseur (178) pour détecter et traiter les données enregistrées dans lesdits moyens de mémoire (98) afin de commander la fonction de transmission des moyens de traitement par le premier chemin de signal (108, 110) en fonction des données extraites desdits moyens de mémoire (98), pour traiter les signaux du capteur et pour fournir les signaux de capteur traités à une borne de sortie (206).

# FIG.1
PRIOR ART

$R_2$   $R_X$   $R_3$

$V_0$

$R_1$   $\underline{10}$   $R_4$

# FIG. 2
PRIOR ART

12

14   13

# FIG. 3

18

# FIG. 4

| R | BINARY CODE VALUE |
|---|---|
| 10 | 0001 |
| 20 | 0010 |
| 30 | 0011 |
| 40 | 0100 |
| 50 | 0101 |

22

1

FIG. 5

FIG. 6

**FIG. 7**

8

70

38

61   62   40

74

72

8

68

**FIG. 8**

68

38

70

74   72

**FIG. 9**

86   80

82

84

88

92

90

FIG. 10

0 079 086

4

FIG. 11

**FIG. 12**

0 079 086